# EUROPÄISCHE PATENTSCHRIFT

(11) **EP 0 831 947 B1**
(45) Veröffentlichungstag und Bekanntmachung des Hinweises auf die Patenterteilung: **18.09.2002**
(21) Anmeldenummer: 96906691.9
(22) Anmeldetag: 02.04.1996
(51) Int. Cl.: A61M 5/315

(54) **INJEKTIONSGERÄT**
INJECTION DEVICE
DISPOSITIF D'INJECTION

(43) Veröffentlichungstag der Anmeldung: 01.04.1998
(73) Patentinhaber: Disetronic Licensing AG, 3400 Burgdorf (CH)
(72) Erfinder: KIRCHHOFER, Fritz, CH-3454 Sumiswald (CH); MICHEL, Peter, CH-3400 Burgdorf (CH)
(74) Vertreter: Schwabe - Sandmair - Marx
(86) Internationale Anmeldenummer: CH9600115
(87) Internationale Veröffentlichungsnummer: WO97036625

(56) Entgegenhaltungen:
- EP-A- 0 373 321
- WO-A-95/04563
- FR-A- 2 701 211

## Beschreibung

Die Erfindung betrifft ein Injektionsgerät zum Injizieren einer vorwählbaren Dosis einer flüssigen Substanz aus einer in einem Ampullenhalter befindlichen Ampulle mit einem mit diesem mechanisch verbundenen hülsenförmigen Mechanikhalter, in dessen Innern eine längsverschiebbare Schubstange mit einem daran angeordneten Stopfen vorgesehen ist, der auf die Ampulle einwirkt, wobei die Schubstange von einer mit dieser mechanisch gekoppelten, ebenfalls längsverschiebbaren Vorschubhülse umgeben ist, die in ihrem oberen Endbereich von einem Deckel abgeschlossen ist.

Ein derartiges Injektionsgerät ist aus der EP 0 373 321 B 1 bekannt, das aus einer Ampullenhülse zur Aufnahme der Ampulle und einer Abgabemechanik besteht. Diese wiederum setzt sich zusammen aus einer zylindrischen Kolbenstange mit einem inneren Hohlzylinder und einem mit diesem einstückig ausgebildeten äusseren Hohlzylinder, der an seiner äusseren Mantelfläche einen Führungsnocken aufweist. Auf dem äusseren Hohlzylinder ist ein Dosierring radial fest angebracht. Mittels einer am äusseren Umfang des Dosierrings angebrachten Skalierung kann durch ein im Gehäuse des Mechanikhalters eingelassenes Sichtfenster die Dosierung abgelesen werden. Die Abgabemechanik ist mittels einer Rückhaltefeder längsbeweglich im Mechanikhalter angeordnet und durch einen mit dem Gehäuse des Mechanikhalters verschraubbaren Verschlussdeckel gesichert. Der Dosierring liegt mit der Kraft der Rückhaltefeder gegen den Verschlussdeckel an. Das Gehäuse des Mechanikhalters ist auf seiner Innenseite mit Längsschlitzen unterschiedlicher Länge versehen, in welcher der entsprechende Führungsnocken der Abgabemechanik gleiten kann. Der Hub der Abgabemechanik wird dadurch von der Länge des mit dem Führungsnocken zusammenwirkenden Längsschlitzes bestimmt. Die Anpassung der zu verabreichenden Dosis an die Bedürfnisse eines Patienten erfolgt ausschliesslich durch eine autorisierte Person wie z.B. einen Arzt. Dazu ist ein Spezialschlüssel notwendig, mittels dessen ein Verriegelungsring des Geräts gelöst werden kann, bis der Führungsnocken der Abgabemechanik aus dem jeweiligen Längsschlitz ausrastet. Der Arzt kann nun durch Rotation der ausgerasteten Abgabemechanik und erneutes Einrasten des Führungsnockens in einen entsprechenden Längsschlitz die gewünschte Dosis auswählen.

Die einmal gewählte Dosis kann nicht ohne erheblichen Aufwand variiert werden. Ausserdem kann aufgrund der Konstruktion der Abgabemechanik das Injektionsgerät nur für eine einmalige Dosisabgabe verwendet werden, obwohl noch möglicherweise ein genügender Medikamentevorrat in der Ampulle vorhanden ist. Um eine weitere Medikamentendosis zu verabreichen, muss die Ampullenhülse vom Mechanikhalter gelöst und zusammen mit der möglicherweise noch Medikamente enthaltenden Ampulle entsorgt werden.

Aus der EP 0 037 696 B 1 ist ein Injektionsgerät gattungsmässiger Art bekannt, bei dem über einen in eine Richtung wirkenden Schubübertragungsmechanismus bei Betätigung eines Kappenverschlusses eine zu verabreichende Dosis einer flüssigen Substanz aus einer Ampulle entnommen werden kann. Der Schubübertragungsmechanismus besteht darin, dass ein Schubteil mit einer Vielzahl von Zähnen und das andere Schubteil mit einer Sperrklinke ausgerüstet sind, die ineinandergreifen und somit den Vorschub des Schubübertragungsmechanismus nur in eine Richtung des Ausstosses der zu verabreichenden Dosis wirken lassen. Über eine Nut und einen Anschlag ist die maximale Hubhöhe und damit das Maximum der zu verabreichenden Dosis begrenzt. Der Bediener des Injektionsgeräts kann auch weniger als die maximale Dosis wählen, indem er auf die Anzahl der Klicks hört, die entstehen, wenn beim Vorschub die Zähne des Schubteils mit der Sperrklinke einrasten. Ein Klicken entspricht dabei der minimalen Dosis. Das Bedienelement, der Kappenverschluss, wird mittels einer Feder zurückgeschoben bis zu einem Anschlag.

Dieses Injektionsgerät ist auf eine äusserst sorgfältig Bedienung ausgelegt, da ein Klicken leicht überhört werden kar und verschiedene zu verabreichende Dosen nicht gesichert eingestellt werden können. Ausserdem ist keine Anzeigeein richtung für die zu wählende Dosis vorgesehen.

Die gattungsbildende FR-A-2 701 211 offenbart ein Injektionsgerät zum Injizieren einer vorwählbaren Dosis einer flüssigen Substanz, mit einem hülsenförmigen Mechanikhalter, in dessen Innenraum eine längsverschiebbare Schubstange vorgesehen ist, die auf eine Ampulle einwirkt. Zum Verabreichen der Dosis wird die Vorschubhülse hinuntergedrückt und dabei gleichzeitig aufgrund einer Verrastung die Schubstange niedergedrückt. Die Vorschubhülse dient gleichzeitig der Dosierung. Hierzu sind in der Umfangswand des Mechanikhalters unterschiedlich lange Längsschlitze vorgesehen, in die ein an der Vorschubhülse angebrachter Nocken eingreift. Zum Verstellen der Dosis muss der Nocken einwärts gedrückt werden und die Vorschubhülse verdreht werden.

Aufgabe der vorliegenden Erfindung ist es, das gattungsgemäße Injektionsgerät dahingehend weiterzubilden, dass der Benutzer die zu verabreichende Dosis noch bediensicherer vorwählen kann.

Diese Aufgabe wird gelöst durch ein Injektionsgerät mit den Merkmalen nach Patentanspruch 1. Vorteilhafte Weiterbildungen sind Gegenstand der rückbezogenen Ansprüche.

Somit ist zum Vorwählen einer zu injizierenden Dosis der flüssigen Substanz eine Dosierhülse mit einem Treppenglied vorgesehen, die am Mechanikhalter in dessen oberen Endbereich angeordnet ist, wobei durch eine Drehbewegung der Dosierhülse mit dem Treppenglied die jeweils zu verabreichende Dosis einstellbar ist.

Die Dosierhülse ist dabei zusammengesetzt aus einem Bedienelement mit einer Profilierung, einem daran anschliessenden Zylinder mit Dosisaufdruck, einem Einrastanschlag und dem daran sich anschliessenden Treppenglied. Die konstruktive Anbindung der Dosierhülse an das Injektionsgerät ist dadurch gekennzeichnet, dass sie zusammen mit dem Treppenglied und dem Zylinder mit dem Dosisaufdruck die Vorschubhülse umgibt.

Das erfindungsgemässe Injektionsgerät hat den wesentlichen Vorteil, dass eine einmal eingestellte Dosis immer wieder appliziert werden kann; der Benutzer muss vor jeder Injektion das Injektionsgerät nur noch laden. Die Vorwahl der zu applizierenden Dosis ist denkbar einfach durch eine einfache Drehbewegung der Dosierhülse.

Weiter vorteilhafte Ausgestaltungen der Erfindung ergeben sich aus den abhängigen Ansprüchen.

Ein Ausführungsbeispiel der Erfindung ist in den Figuren dargestellt.

Es zeigen:
Fig. 1: das erfindungsgemässe Injektionsgerät im Schnitt;
Fig. 2: die Dosierhülse mit Treppenglied in einer perspektivischen Ansicht;
Fig. 3: ein Schnitt längs der Linie A - A in Figur 1.

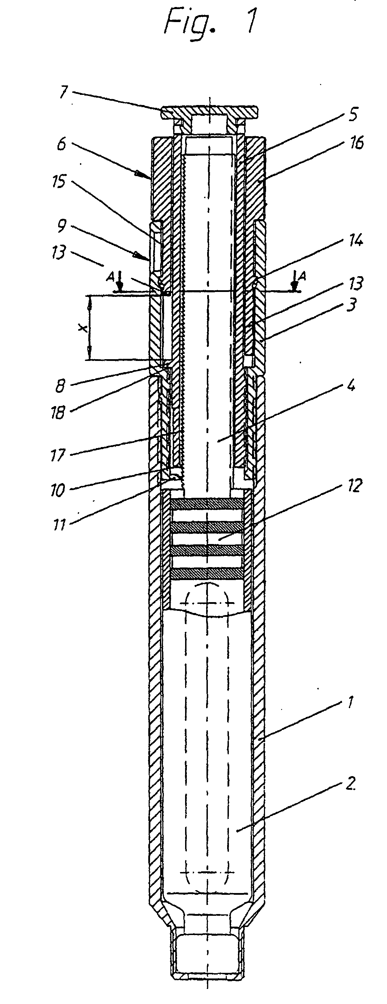

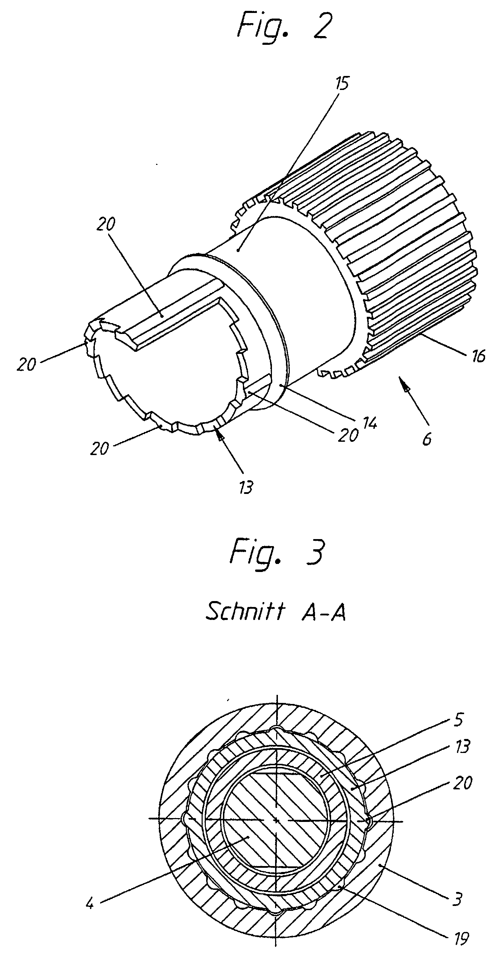

Das in Fig. 1 gezeigte Injektionsgerät besteht aus dem Ampullenhalter 1, in dem sich die Ampulle 2 mit dem Stopfen 12 und mit der zu verabreichenden flüssigen Substanz befindet. Mit dem Ampullenhalter 1 ist der hülsenförmige Mechanikhalter 3 durch ein Gewinde oder einen anderen geeigneten Verschluss, z.B. Bajonett-Verschluss, mechanisch verbunden. Im Innern des Mechanikhalters 3 ist die längsverschiebbare Schubstange 4 angeordnet, die auf den Stopfen 12 der Ampulle 2 einwirkt. Die Schubstange 4 ist von der mit dieser mechanisch gekoppelten, ebenfalls längsverschiebbaren Vorschubhülse 5 umgeben, die in ihrem oberen Endbereich von dem Deckel 7 abgeschlossen ist. Zum Vorwählen einer zu injizierenden Dosis der flüssigen Substanz ist die Dosierhülse 6 mit dem Treppenglied 13 vorgesehen, die am Mechanikhalter 3 in dessen oberen Endbereich angeordnet ist. Dabei umgibt die Dosierhülse 6 mit dem Treppenglied 13 die Vorschubhülse 5. Durch eine einfache Drehbewegung der Dosierhülse 6 mit dem Treppenglied 13 ist die jeweils einzustellende und zu verabreichende Dosis vorwählbar. Die Dosierhülse 6 ist über den Einrastanschlag 14 mit dem Mechanikhalter 3 mechanisch gekoppelt. Die Dosierhülse 6 besteht gemäss Fig. 2 aus dem profilierten Bedienelement 16, dem daran sich anschliessenden Zylinder 15 mit dem Dosisaufdruck, dem Einrastanschlag 14 und dem sich daran anschliessenden Treppenglied 13. An der Vorschubhülse 5 ist der Nocken 8 angeordnet, der beim Herausziehen der Vorschubhülse 5 in Richtung des Deckels 7 gegen die jeweils eingestellte Treppenstufe des Treppenglieds 13 anschlägt. Beim Niederdrücken der Vorschubhülse 5 schlägt der Nocken 8 gegen die Schulter 18 des Mechanikhalters 3 an, wodurch sich der Injektionshub X ergibt.

Zur mechanischen Kopplung von Schubstange 4 mit der Vorschubhülse 5 ist die Schubstange 4 mit der Verzahnung 17 versehen, die mit dem Rastnocken 10 der Vorschubhülse 5 zusammenwirkt. Dabei entspricht die Zahnhöhe der Zähne der Verzahnung 17 an der Schubstange 4 einer Stufenhöhe des Treppenglieds 13. Der Mechanikhalter 3 weist im Bereich seines unteren Endes die Raste 11 auf, die ebenfalls mit der Verzahnung 17 der Schubstange 4 zusammenwirkt. Der Dosisaufdruck auf dem Zylinder 15 ist über das Fenster 9 im Mechanikhalter 3 sichtbar. Der Mechanikhalter 3 weist in seinem Innern eine Vielzahl von Ausbuchtungen 19 auf, in die die Vorsprünge 20 des Treppenglieds 13 einrasten. (Fig. 3) Die Schubstange 4 ist drehfest längsverschiebbar.

Die Funktionsweise des erfindungsgemässen Injektionsgeräts ist folgende:

Der Bediener dreht die Dosierhülse 6 mit dem Treppenglied 13 solange, bis der gewünschte Dosisaufdruck auf dem Zylinder 15 im Fenster 9 erscheint. Dann wird das Injektionsgerät geladen. Fig. 1 zeigt dieses vor dem Laden. Zum Laden wird die Vorschubhülse 5 durch den mit ihr mechanisch verbundenen Deckel 7 nach oben gezogen, bis der Nocken 8, der Bestandteil der Vorschubhülse 5 ist, gegen die aktuell eingestellte Treppenstufe des Treppenglieds 13 anschlägt. Dabei gleitet der Rastnocken 10 der Vorschubhülse 5 über die Verzahnung 17 der Schubstange 4, deren Zähne (nicht dargestellt) nach unten gepfeilt sind. Um zu verhindern, dass die Schubstange 4 dennoch dabei nach oben geschoben wird, ist die Raste 11 am Mechanikhalter 3 vorgesehen, der ja nicht längsverschiebbar ist. Somit stemmt sich die Raste 11 in die Verzahnung 17 und verhindert eine Bewegung nach oben der Schubstange 4. Zum Verabreichen der vorgewählten Dosis wird vom Bediener Druck auf den Deckel 7 und auf die mit ihm verbundene Vorschubhülse 5 in Richtung nach unten ausgeübt. Dabei stemmt sich der Rastnocken 10 in die Verzahnung 17 und nimmt die Schubstange 4 quasi mit und der mit der Schubstange 4 verbundene Stopfen 12 übt Druck auf die im Ampullenhalter 1 befindliche Ampulle 2 aus, wodurch die flüssige Substanz appliziert wird, indem sie durch eine nicht dargestellte Injektionsnadel verabreicht wird. Beim Niederdrücken der Vorschubhülse 5 mittels des Deckels 7 gleiten die Zähne der Verzahnung 17 auch über die Raste 11 des Mechanikhalters 3, wobei diese durch die gewählte Pfeilung der Zähne der Verzahnung 17 ausser Eingriff ist. Die Vorschubbewegung der Vorschubhülse 5 in Richtung nach unten dauert solange an, bis der Nocken 8 gegen die Schulter 18 des Mechanikhalters 3 anschlägt, so dass die vorgewählte Dosis der flüssigen Substanz verabreicht ist. Durch erneutes Laden ist das Injektionsgerät für einen oder mehrere nachfolgende Injektionsvorgänge bereit. Die Schubstange 4 verharrt bei den Ladevorgängen im unmittelbar zuvor gegebenen Zustand durch die Verzahnung von Raste 11 mit den Zähnen der Verzahnung 17. Insbesondere bewegt sich die Schubstange nur in eine Richtung, nämlich nach unten, während die Vorschubhülse sich in zwei Richtungen bewegen lässt, nämlich zum Laden nach oben und zum Applizieren nach unten. Das erfindungsgemässe Injektionsgerät kann zum Verabreichen von Medikamenten dienen. Es können aber auch Lotionen und andere flüssige Substanzen damit verabreicht werden.

Die Länge des Injektionshubs X ist durch die einzelnen Differenzen der Treppenstufen des Treppenglieds 13 so festgelegt, dass bei einer Ampulle 2 mit einem bestimmten Durchmesser, X ein ganzzahliges Vielfaches einer Dosiseinheit ist.

## Patentansprüche

1. Injektionsgerät zum Injizieren einer vorwählbaren Dosis einer flüssigen Substanz aus einer in einem Ampullenhalter (1) befindlichen Ampulle (2) mit einem Stopfen (12) und mit einem mit diesem mechanisch verbundenen hülsenförmigen Mechanikhalter (3), in dessen Innern eine längsverschiebbare Schubstange (4) vorgesehen ist, die auf die Ampulle (2) einwirkt, wobei die Schubstange (4) von einer mit dieser mechanisch gekoppelten, ebenfalls längsverschiebbaren Vorschubhülse (5) umgeben ist, wobei zum Vorwählen einer zu injizierenden Dosis der flüssigen Substanz an der Vorschubhülse (5) ein Nocken (8) angeordnet ist, der beim Herausziehen der Vorschubhülse (5) aus dem Mechanikhalter (3) gegen einen oberen Anschlag anschlägt und beim Niederdrücken der Vorschubhülse (5) gegen eine Schulter (18) des Mechanikhalters anschlägt, **dadurch gekennzeichnet, dass** im oberen Endbereich des Mechanikhalters (3) eine Dosierhülse (6), die aufgrund einer mechanischen Kopplung mit diesem nicht längsverschiebbar ist, mit einem Treppenglied (13) vorgesehen ist, wobei durch eine Drehbewegung der Dosierhülse (6) mit dem Treppenglied (13) eine der Treppenstufen des Treppenglieds als der obere Anschlag eingestellt wird, um die jeweils zu verabreichende Dosis einzustellen.

2. Injektionsgerät nach Anspruch 1, **dadurch gekennzeichnet, dass** die Dosierhülse (6) mit dem Treppenglied (13) die Vorschubhülse (5) umgibt.

3. Injektionsgerät nach Anspruch 1 oder 2, **dadurch gekennzeichnet, dass** zur mechanischen Kopplung von Schubstange (4) mit Vorschubhülse (5) die Schubstange (4) mit einer Verzahnung (17) versehen ist, die mit einem Rastnocken (10) der Vorschubhülse zusammenwirkt.

4. Injektionsgerät nach einem der Ansprüche 1 bis 3, **dadurch gekennzeichnet, dass** die Zahnhöhe der Zähne der Verzahnung (17) an der Schubstange (4) einer Stufenhöhe des Treppenglieds (13) entspricht.

5. Injektionsgerät nach einem der Ansprüche 1 bis 4, **dadurch gekennzeichnet, dass** der Mechanikhalter (3) im Bereich seines unteren Endes eine Raste (11) aufweist, die mit der Verzahnung (17) der Schubstange (4) zusammenwirkt.

6. Injektionsgerät nach einem der Ansprüche 1 bis 5, **dadurch gekennzeichnet, dass** die Dosierhülse (6) zusammengesetzt ist aus einem Bedienelement (16) mit einer Profilierung, einem daran anschließenden Zylinder (15) mit Dosisaufdruck, einem Einrastanschlag (14) und dem daran sich anschließenden Treppenglied (13).

7. Injektionsgerät nach einem der Ansprüche 1 bis 6, **dadurch gekennzeichnet, dass** der Dosisaufdruck auf dem Zylinder (15) über ein Fenster (9) im Mechanikhalter (3) sichtbar ist.

8. Injektionsgerät nach einem der Ansprüche 1 bis 5, **dadurch gekennzeichnet, dass** der Mechanikhalter (3) in seinem Innern eine Vielzahl von Ausbuchtungen (19) aufweist, in die Vorsprünge (20) des Treppenglieds (13) einrasten.

## Claims

1. Injection device for injecting a dose - which can be pre-selected - of a liquid substance out of an ampoule (2) located in an ampoule holder (1), with a plug (12) and a barrel-shaped mechanism holder (3) which is mechanically connected to it and in whose interior a sliding rod (4) is located which can be pushed longitudinally and which acts on the ampoule (2), wherein the sliding rod (4) is enclosed by an advance feed barrel (5) which is mechanically coupled to it and can likewise be pushed longitudinally, wherein in order to pre-select a dose of the liquid substance which is to be injected, located on the advance feed barrel (5) is a cam (8) which strikes against an upper limit stop when the advance feed barrel (5) is pulled out of the mechanism holder (3), and strikes against a shoulder (18) of the mechanism holder when the advance feed barrel (5) is pressed down,
**characterised in that**
in the upper end area of the mechanism holder (3), a dosing barrel (6) which cannot be pushed longitudinally on account of a mechanical coupling with it, is provided with a step element (13), wherein through a rotational movement of the dosing barrel (6) with the step element (13), one of the step stages of the step element is set as the upper limit stop, in order to set the dose which is to be dispensed in the respective case.

2. Injection device in accordance with claim 1, **characterised in that** the dosing barrel (6) with the step element (13) surrounds the advance feed barrel (5).

3. Injection device in accordance with claim 1 or 2, **characterised in that** for the mechanical coupling of the sliding rod (4) with the advance feed barrel (5), the sliding rod (4) is equipped with toothing (17) which interacts with a snap-in cam (10) of the advance feed barrel.

4. Injection device in accordance with one of the claims 1 to 3, **characterised in that** the tooth height of the teeth in the toothing (17) on the sliding rod (4) corresponds to one step height of the step element (13).

5. Injection device in accordance with one of the claims 1 to 4, **characterised in that** in the area of its lower end, the mechanism holder (3) has a stop notch (11) which interacts with the toothing (17) of the sliding rod (4).

6. Injection device in accordance with one of the claims 1 to 5, **characterised in that** the dosing barrel (6) comprises an operating element (16) with a profile, a cylinder (15) which follows on from that and which has dosages printed on, a snap-in limit stop (14), and the step element (13) which joins on to that.

7. Injection device in accordance with one of the claims 1 to 6, **characterised in that** the dosage imprint on the cylinder (15) is visible via a window (9) in the mechanism holder (3).

8. Injection device in accordance with one of the claims 1 to 5, **characterised in that** in its interior, the mechanism holder (3) has numerous dents (19) into which the projections (20) of the step element (13) engage.

## Revendications

1. Dispositif d'injection pour injecter une dose pré-sélectionnable d'une substance fluide à partir d'une ampoule (2) située dans un porte-ampoule (1), comprenant un bouchon (12) et une pièce mécanique de retenue (3) en forme de douille, qui est reliée mécaniquement audit bouchon et à l'intérieur de laquelle est prévue une tige de poussée (4) pouvant coulisser longitudinalement et agissant sur l'ampoule (2), sachant que la tige de poussée (4) est entourée par une douille d'avance (5) pouvant semblablement coulisser dans le sens longitudinal et accouplée mécaniquement avec ladite tige, la douille d'avance (5) présentant, en vue de la présélection d'une dose de la substance fluide devant être injectée, un ergot (8) venant se plaquer contre une butée supérieure lors de l'extraction de la douille d'avance (5) hors de la pièce mécanique de retenue (3), et venant buter contre un épaulement (18) de ladite pièce mécanique de retenue lors de l'enfoncement de ladite douille d'avance (5), **caractérisé par le fait qu'**il est prévu, dans la région extrême supérieure de la pièce mécanique de retenue (3), une douille de dosage (6) qui ne peut pas coulisser longitudinalement avec cette dernière du fait d'un accouplement mécanique, et est pourvue d'une pièce étagée (13), dispositif dans lequel, suite à un mouvement rotatoire de la douille de dosage (6) avec la pièce étagée (13), l'un des gradins de ladite pièce étagée est ajusté en tant que butée supérieure de manière à régler la dose devant être respectivement administrée.

2. Dispositif d'injection selon la revendication 1, **caractérisé par le fait que** la douille de dosage (6) entoure la douille d'avance (5) par la pièce étagée (13).

3. Dispositif d'injection selon la revendication 1 ou 2, **caractérisé par le fait que**, en vue de l'accouplement mécanique de la tige de poussée (4) avec la douille d'avance (5), ladite tige de poussée (4) est munie d'une denture (17) coopérant avec un mentonnet d'encliquetage (10) de ladite douille d'avance.

4. Dispositif d'injection selon l'une des revendications 1 à 3, **caractérisé par le fait que** la hauteur des dents de la denture (17), sur la tige de poussée (4), correspond à une hauteur de gradin de la pièce étagée (13).

5. Dispositif d'injection selon l'une des revendications 1 à 4, **caractérisé par le fait que** la pièce mécanique de retenue (3) présente, dans la région de son extrémité inférieure, un cran (11) coopérant avec la denture (17) de la tige de poussée (4).

6. Dispositif d'injection selon l'une des revendications 1 à 5, **caractérisé par le fait que** la douille de dosage (6) est composée d'un élément d'actionnement (16) possédant un profilage, d'un cylindre (15) attenant audit élément et présentant une graduation de dosage, d'une butée (14) d'encliquetage extrême, et de la pièce étagée (13) attenante à cette dernière.

7. Dispositif d'injection selon l'une des revendications 1 à 6, **caractérisé par le fait que** la graduation de dosage, inscrite sur le cylindre (15), est visible à travers une fenêtre (9) pratiquée dans la pièce mécanique de retenue (3).

8. Dispositif d'injection selon l'une des revendications 1 à 5, **caractérisé par le fait que** la pièce mécanique de retenue (3) comprend, dans son espace intérieur, un grand nombre d'indentations (19) dans lesquelles s'encliquettent des protubérances (20) de la pièce étagée (13).
